# EUROPEAN PATENT SPECIFICATION

(11) **EP 2 567 666 B1**
(45) Date of publication and mention of the grant of the patent: **31.03.2021**
(21) Application number: 11007282.4
(22) Date of filing: 07.09.2011
(51) Int. Cl.: A61B 17/16, A61C 1/12, A61B 90/30

(54) **Surgical power tool**
Chirurgisches Elektrowerkzeug
Outil chirurgical électrique

(43) Date of publication of application: 13.03.2013
(60) Divisional application: 13002903.6
(73) Proprietor: Stryker European Holdings I, LLC, Kalamazoo, MI 49002 (US)
(72) Inventor: Schmuck, Manfred, 78570 Mühlheim-Stetten / Donau (DE); Greiner, Karl, 78570 Mühlheim (DE); Knöpfle, Christian, 78166 Donaueschingen (DE)
(74) Representative: Röthinger, Rainer

(56) References cited:
- EP-A2- 2 263 568
- WO-A1-2006/031184
- WO-A1-2011/007351
- JP-A- 2011 167 796
- US-A1- 2009 088 770
- US-A1- 2010 107 423
- US-A1- 2011 006 101

## Description

### Technical Field

The present disclosure relates to a surgical power tool with a head portion having a tool and a shaft which is connected to the head portion for guiding the head portion to a surgical site.

### Background

Surgical procedures often involve drilling, screwing or other operations at a bone. Surgical power tools are known in the art which support the surgeon when such operations need to be performed.

EP 2 263 568 A2 discloses a surgical stapler having a handle which is connected to a remote motorized staple assembly by a highly flexible cable. Further, a surgical instrument is provided having a pair of grasping jaws to grasp and hold a housing of the surgical stapler for guiding the stapler to a tissue to perform a stapling procedure.

WO 2011/007351 A1 discloses a surgical stapler having a mechanical drive cable assembly for the stapler mechanism separated spatially from a lockable joint used to align and grasp the stapler in its desired position.

WO 2006/031184 A1 discloses a handle comprising an elongate tubular sleeve intended to surround a corresponding tool part. The sleeve is connected to a hand-filling body via a flexible elongate element that has a first of the ends thereof connected to the body and the second end thereof connected to the sleeve.

US 2011/0006101 A1 discloses a surgical fastener apparatus which comprises a handle and an elongated shaft having a proximal end attached to the handle and a distal end extending therefrom. The apparatus further comprises an end effector including a pair of jaws pivoted at a proximal end thereof and movable between an open and closed position.

An angled surgical driver for use in surgical procedures is described in document US 2009/088770 A1. The driver includes a shaft with an elongated shape for accessing locations within the patient. The shaft includes a first section and a second section that may be positioned at various relative angles depending upon the context of use. A mount is positioned at an end of the shaft to receive a variety of tool bits. A drive mechanism extends through the shaft to provide rotation to the mount and engaged tools. The tool bits may include various drive ends including hexagonal, Torx, Philips, flathead, in addition to various sizes of drills. A handle extends outward from the first section. The handle is shaped and sized to facilitate grasping by a surgeon.

Document US 2010/107423 A1 discloses a hand-held power tool comprising a housing having a primary handle mounting surface and a secondary handle mounting feature consisting of a pair of bores disposed on opposite sides of the housing, a motor disposed in the housing, a drive spindle disposed in the housing and coupled to the motor, a primary handle fixedly coupled to the primary handle mounting surface, a first secondary handle having a pair of spaced apart legs operable to be selectively attached to the pair of bores at the opposite sides of the housing, the first secondary handle being a bail handle, and a second secondary handle having a single attachment feature operable to selectively attach the secondary handle to the housing at either one of the bores, the second secondary handle being a side handle.

From document JP 2011 167796 a powered tool with a clampable side handle is known.

A surgical power tool is, for example, disclosed in EP 1 836 976 A1. The power tool comprises a drill or similar tool arranged at a distal end of the power tool and a gripping shaft to handle the power tool. The longitudinal axis of the gripping shaft is angled with respect to the drill or other tool.

When the surgeon accomplishes a surgical procedure, he or she has to exert pressure over the gripping shaft of the surgical power tool, so that enough force will arrive at the distally arranged tool. This situation could result in a slip of, for example, the drill at the surgical site, and the patient being treated can get injured. Consequently, it requires a great effort for the surgeon to carry out an accurate drill or cut with a surgical power tool without injuring the patient.

### Summary

There is a need for a surgical power tool which supports the surgeon to carry out an accurate operation at the surgical site and reduces the risk of injuring the patient.

A surgical tool is provided that comprises a head portion having a tool in the form of a drill or a screw driver blade with a tool axis and a shaft which is connected to the head portion for guiding the head portion to a surgical site, wherein the shaft has a longitudinal axis and the tool axis is angled with respect to the longitudinal axis of the shaft. The surgical power tool further comprises a handle portion for exerting pressure to the head portion along the tool axis. The handle portion comprises a clamping element to releasably secure the handle portion to the shaft and further comprises a handle having a longitudinal axis that is substantially coaxial with the tool axis.

The axis of the tool is angled with respect to the shaft of the surgical power tool. This angle may be defined such that the user can easily reach the desired position at the surgical site. Thus, the angle can be between 60 and 120 degrees, for example between 80 and 100 degrees (e.g., approximately 90 degrees). The angle may be adjustable (e.g., mechanically or electrically).

The power tool can be powered by any kind of electric motor. The electric motor can be accommodated in the shaft or the handle portion or can be connected to the surgical power tool by means of a driveshaft. The transfer of the power to the angled tool can be accomplished by any kind of transmission. The power tool can also be powered by pressurized air or in any other manner.

The handle portion comprises a handle, preferably at its proximal end, the handle for being gripped by a surgeon. The longitudinal axis of the handle is substantially coaxial with the tool axis. There can be any tolerance (e.g., up to 20 degrees) between the longitudinal axis of the handle and the tool axis. Due to the arrangement of the handle and the tool, pressure can be exerted to the head portion. With the longitudinal axis of the handle being coaxial with the tool axis, the pressure can be accurately exerted in the direction of the tool axis and therewith to the surgical site.

In an optional aspect, the handle of the handle portion is angled with respect to the shaft, so that pressure can be exerted in a direction toward the tool axis. The resultant force exerted by the surgeon on the handle may be directed towards the tool axis to support the surgical procedure. The angle between the handle and the shaft can be between 60 and 120 degrees, for example between 80 and 100 degrees (e.g., approximately 90 degrees).

According to a further optional aspect, the handle portion is configured to secure the handle portion to the shaft, so that the handle portion is fixed and cannot be moved or rotated with respect to the shaft. This securing can be accomplished in a releasable manner, for example by the clamping element. The clamping element may be arranged at a distal end of the handle portion and can at least partly encompass the shaft. The clamping element can have a C- or U-like shape to accommodate the shaft. Any other shape is feasible as long as the connection can be fixed.

In an exemplary implementation, the connection is such that the handle portion can easily be removed from the shaft (e.g., so that both parts can be individually sterilized). The handle portion can be secured to the shaft by means of a connecting fastener which can be a locking screw, snap-in connection or interlocking connection.

Advantageously, the clamping element is distanced from the head portion, so that the handle portion is not directly connected to the head portion of the surgical power tool. The clamping element may be connected to the shaft close to the head portion to provide a small lever arm defined between the clamping element and the tool along the shaft.

The clamping element may further be designed such that the handle portion can be adjusted relative to the longitudinal axis of the shaft, for example by a guided rail. The clamping element can also comprise some kind of electric contacts, so that information can be transferred from the handle portion to the shaft or the head portion.

According to another optional aspect, the surgical power tool comprises an illumination device for providing light to the surgical site. The light may be guided by an optical cable to the surgical site or a light source can be arranged directly at the head portion. The light may be cool light.

The illumination device can be guided along or inside the handle portion. In particular, the handle portion may be adapted to accommodate at least one part of the illumination device, for example a power supply of the light source, a switch of the light source and/or the light source itself. The illumination device can also be guided along or inside the shaft.

In one realization, the surgical power tool comprises at least one of a mirror and an endoscope which provides a view of the surgical site, in particular the portion of the site illuminated by a illumination device. The mirror or endoscope can be arranged at the head portion, but it can also be arranged at the shaft or the handle portion.

The mirror or endoscope or at least one part of it may be releasably connected to the surgical power tool. The mirror or endoscope may be adjusted, mechanically or electrically, by for example an electric motor. The adjustment may be provided remotely from the shaft or the handle portion.

According to a further optional aspect, the surgical power tool comprises a rinsing device for providing fluid to the surgical site. The rinsing device can be guided at or inside the shaft or handle portion towards the tool. Any system which can provide a constant stream or flash of fluid can be connected to a rinsing inlet of the rinsing device.

In a further realization, a fixture device may be arranged at the head portion, the shaft or the handle portion to hold any parts which are needed to carry out the surgical procedure. In particular, the fixture device may provide a clamping arrangement to hold the needed parts. These parts can comprise a tissue protection sleeve, a fixation module or plate that is be secured to the bone.

### Brief Description of the Drawings

These and other features, aspects and advantages of the present disclosure will become more apparent from the following detailed description taken in conjunction with the accompanying drawings, wherein:
Fig. 1 shows an embodiment of a surgical power tool in a perspective view;
Fig. 2 is an enlarged perspective view of the surgical power tool showing Fig. 1;
Fig. 3 is a further enlarged perspective view of the surgical power tool showing Fig. 1;
Fig. 4 is a perspective view of a further embodiment of a surgical power tool;
Fig. 5 is an enlarged perspective view of the embodiment shown in Fig. 4; and
Fig. 6 is a further enlarged perspective view of the embodiment shown in Fig. 4.

### Detailed Description

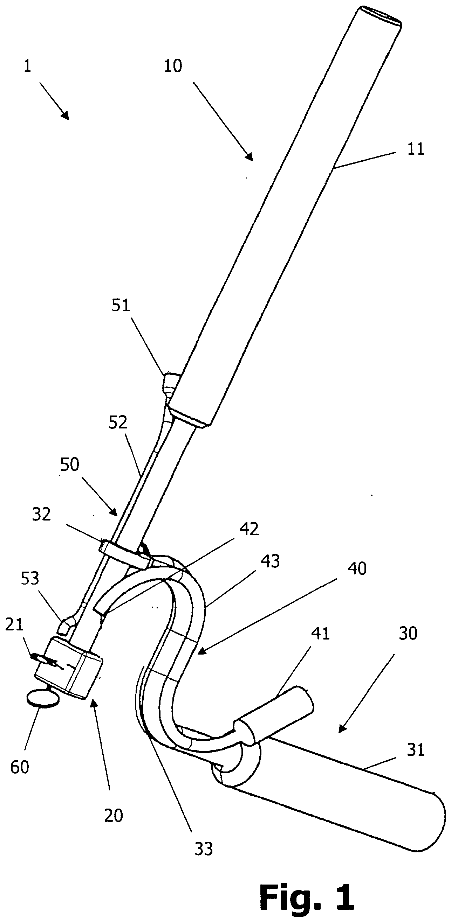

Referring to Fig. 1, there is shown an embodiment of a surgical power tool 1 in the form of a surgical power drill. As shown, the surgical power tool 1 comprises a shaft 10 which is surrounded at its proximal end by a grip 11. The grip 11 extends over approximately at least half of the length of the whole shaft 10, so that a surgeon can easily handle the power tool 1. A head portion 20 is arranged at the distal end of the shaft 10. This head portion 20 carries a tool 21 in the form of a drill to create a bore in the bone at the surgical site. The shaft 10 can comprise components to operate and/or drive the tool 21 of the surgical power tool 1. Moreover, the tool 21 may be removable from the head portion 20.

As illustrated in Fig. 1, the tool 21 as a tool axis that is angled with respect to a longitudinal axis of the shaft 10. In the present embodiment, the angle is 90 degrees and may be adjustable within a predefined range.

The surgical power tool 1 further comprises a handle portion 30 having a handle 31 at its proximal end. The handle 31 is long enough so that at least one hand of the surgeon can easily grasp the handle 31. A longitudinal axis of the handle 31 is parallel to the tool axis of the tool 21.

The handle portion 30 further comprises a linkage 33 and a clamping element 32 at its distal end. The linkage 33 is arranged between the handle 31 and the clamping element 32. The linkage 33 is formed in a bent shape and is rigid, so that application forces can be transferred. Specifically, the linkage 33 has the form of approximately an S to facilitate, for example, intra-oral and/or transbuccal procedures. The linkage 33 is located in a plane defined by a longitudinal axis of the shaft 10 and the longitudinal axis of the handle 31.

The clamping element 32 is arranged at the distal end of the handle portion 30 and is configured to secure the handle portion 30 to the shaft 10. The location of the clamping element 32 is situated on the shaft 10 between the head portion 20 and the grip 11. The clamping element 32 is located spaced apart but near the head portion 20, so that pressure can be exerted to the tool 21 over the clamping element 32 without straddling the linkage 33.

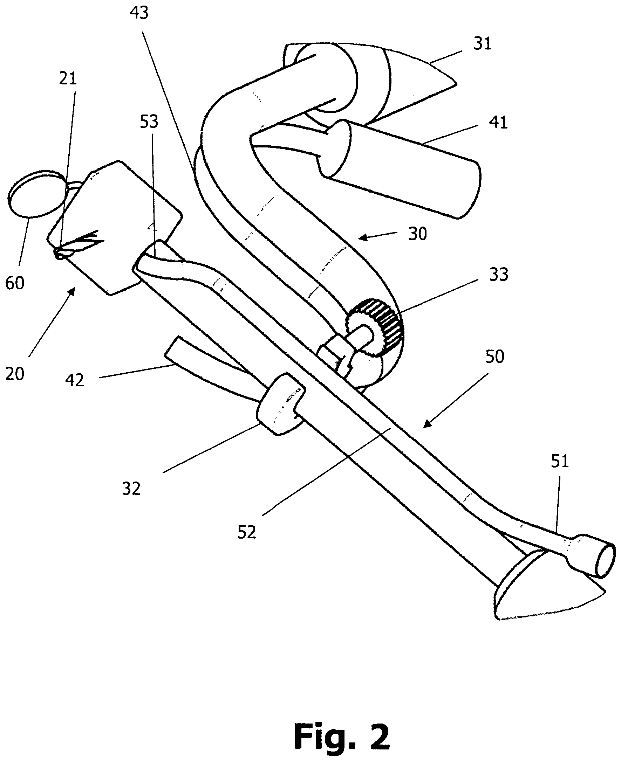

As shown in Fig. 2, the clamping element 32 partly encompasses the shaft 10 and is locked by a connecting fastener 33. The shaft 10 is fixedly clamped by the clamping element 32, so that no movement or rotation of the handle portion 30 relative to the shaft 10 is possible. The connecting fastener 33 is a locking screw which is configured to push one part of the clamping element 32 towards the shaft 10 and therewith provides a frictional engagement. Thus, handle 30 and the shaft 10 are releasably fixed to each other.

A head of the locking screw 34 is large enough so that the surgeon can easily secure and release the locking screw 34. Due to the straight shape of the shaft 10, the clamping element 32 can be axially moved along the shaft 10 to any desired position when the locking screw 34 is released. The handle 31 can also be removed from the shaft 10 for cleaning and sterilization activities.

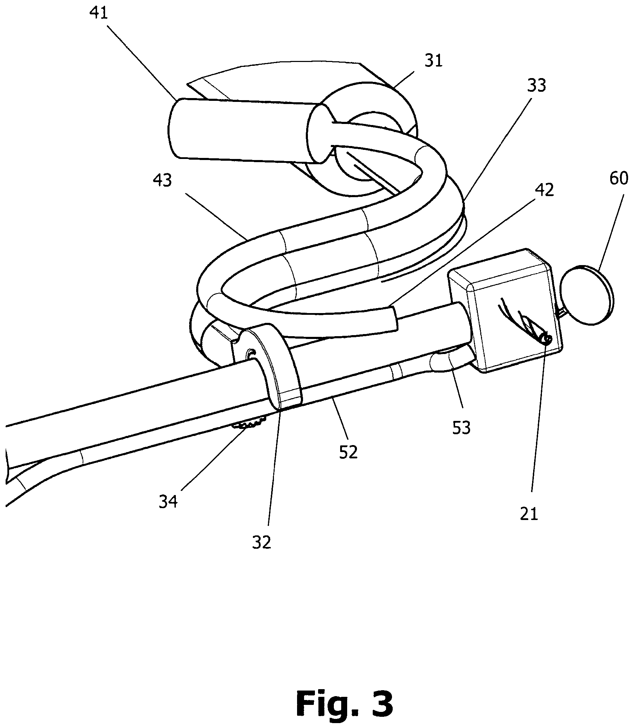

As illustrated in Figs. 2 and 3, an illumination device 40 comprising a light inlet 41, an optical cable 43 and a light outlet 42 is (mainly) arranged along the linkage 33 of the handle portion 30. The light inlet 41 comprises a connector (not shown) to receive a light unit which generates a beam of cool light. The light outlet 42 is directed toward the tool 21 to illuminate the surgical site, wherein the light outlet 42 can be movable to position the light outlet 42 such that the surgeon is not bothered.

The surgical power tool 1 further comprises a rinsing device 50 having a rinsing inlet 51, a tube 52 and a rinsing outlet 53. The rinsing inlet 51 is configured to be coupled to any device capable of providing a rinsing fluid. The tube 52 leads the fluid from the rinsing inlet 51 to the rinsing outlet 53. The rinsing outlet 53 can be selectively positioned by the surgeon such that the fluid is directed towards the surgical site. The rinsing device 50 is guided along and connected to the part of the shaft 10 which is not encompassed by the grip 11.

A mirror 60 is arranged at the head portion 20 opposite of the shaft 10, so that the surgeon operating the surgical power tool 1 can view the surgical site through this mirror 60. The mirror 60 can be adjusted as needed by the surgeon. In addition or as an alternative to the mirror 60, an endoscope may be used to provide a view of the surgical site. The endoscope may be attached to the surgical power tool 1 in a similar manner as (e.g., instead of) the illumination device 40 and the rinsing device 50.

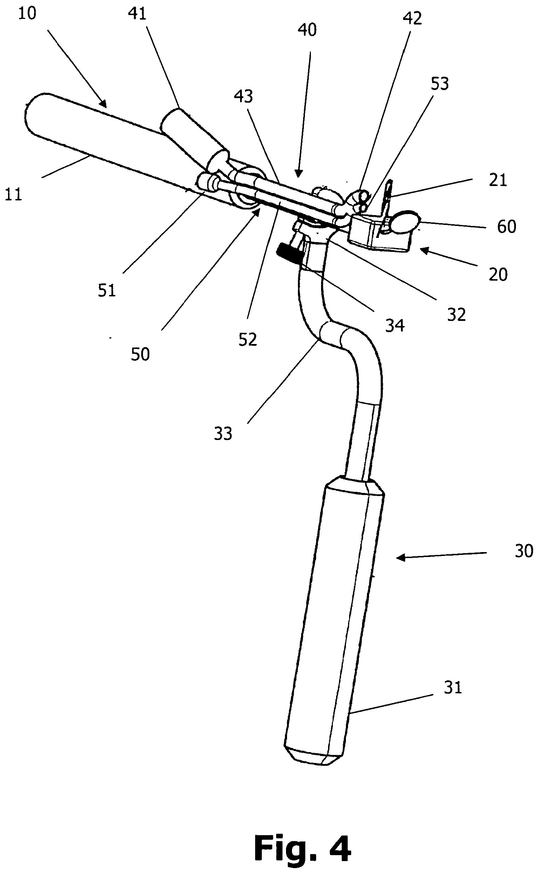

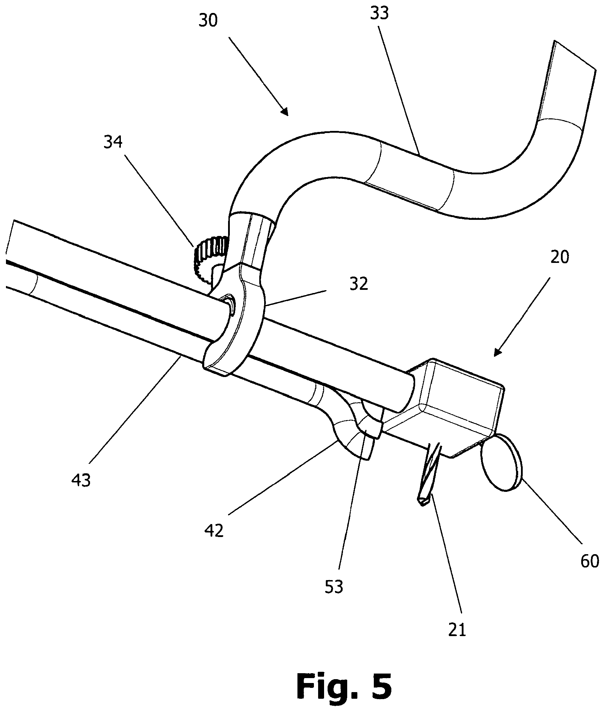

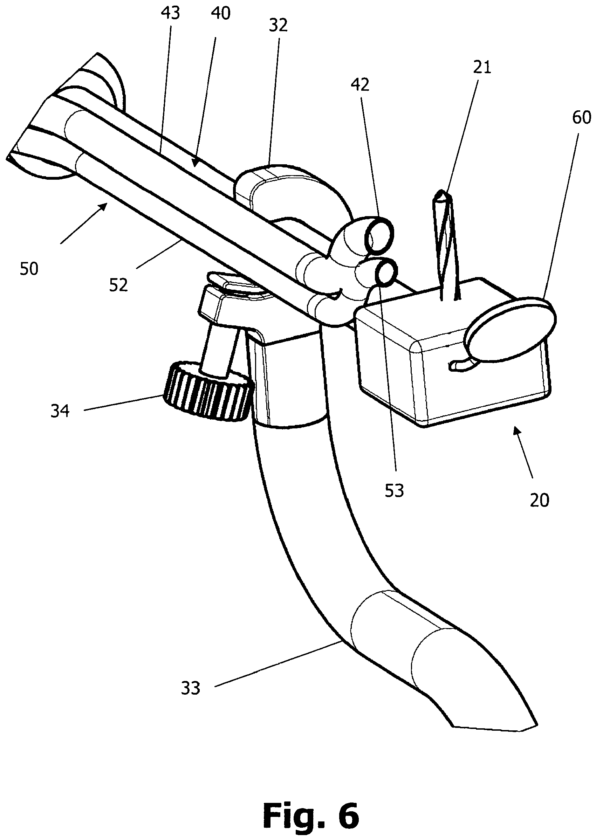

Figs. 4 to 6 show a further embodiment of a surgical power tool 1. The difference between this embodiment and the aforementioned embodiment is that the illumination device 40 is now arranged at the shaft 10 next to the rinsing device 50. All other parts and features correspond to the aforementioned embodiment.

As has become apparent from the embodiments, the shaft for guiding the head portion to the surgical site and the handle portion to exert pressure to the tool are separated from each other, so that the surgeon can accurately guide the head portion to the desired position with one hand and can further exert pressure to the handle portion with the other hand.

The surgical power tool described in the above embodiments has a tool in the form of a drill. It will be appreciated that the tool could alternatively be realized by a screw driver blade or in any other manner.

While the present disclosure has been described with respect to particular embodiments, those skilled in the art will recognize that the present invention is not limited to the specific embodiments described and illustrated herein. It is to be understood that the disclosure is only illustrative. Accordingly, it is intended that the invention be limited only by the scope of the claims attended hereto.

## Claims

1. A surgical power tool (1) comprising
- a head portion (20) having a tool (21) in form of a drill or a screw driver blade with a tool axis,
- a shaft (10) which is connected to the head portion (20) for guiding the head portion (20) to a surgical site, the shaft (10) having a longitudinal axis and the tool axis being angled with respect to the longitudinal axis of the shaft (10), and
- a handle portion (30) for exerting pressure to the head portion (20) along the tool axis,
the handle portion (30) comprising a clamping element (32) to releasably secure the handle portion (30) to the shaft (10),
**characterized in that**
the handle portion (30) comprises a handle (31) having a longitudinal axis that is substantially coaxial with the tool axis.

2. The surgical power tool according to claim 1, wherein the tool axis is angled with an angle of 60 to 120 degrees with respect to the longitudinal axis of the shaft (10).

3. The surgical power tool according to one of the preceding claims, wherein the clamping element (32) is secured to the shaft (10) at a distance from the head portion (20).

4. The surgical power tool according to one of the preceding claims, wherein the handle portion (30) is adjustable relative to the longitudinal axis of the shaft (10).

5. The surgical power tool according to one of the preceding claims, wherein the surgical power tool (1) further comprises an illumination device (40) for providing light to the surgical site.

6. The surgical power tool according to claim 5, wherein the handle portion (30) is adapted to accommodate at least one part of the illumination device (40).

7. The surgical power tool according to one of the preceding claims, wherein the surgical power tool (1) further comprises at least one of a mirror (60) and an endoscope configured to provide a view of the surgical site.

8. The surgical power tool according to claim 7, wherein the mirror (60) and/or endoscope are/is arranged at the head portion (20).

9. The surgical power tool according to one of the preceding claims, wherein the surgical power tool (1) further comprises a rinsing device (50) for providing fluid to the surgical site.

10. The surgical power tool according to claim 9, wherein the rinsing device (50) is arranged at least partly along at least one of the shaft (10) and the handle portion (30).

## Patentansprüche

1. Chirurgisches Elektrowerkzeug (1), das umfasst:
ein Kopfteil (20), das ein Werkzeug (21) in Form eines Bohrers oder einer Schraubendreherklinge mit einer Werkzeugachse aufweist,
einen Schaft (10), der mit dem Kopfteil (20) verbunden ist, um das Kopfteil (20) zu einer chirurgischen Eingriffsstelle zu führen, wobei der Schaft (10) eine Längsachse aufweist und die Werkzeugachse in Bezug auf die Längsachse des Schafts (10) abgewinkelt ist, und
ein Griffteil (30), um entlang der Werkzeugachse Druck auf das Kopfteil (20) auszuüben,
wobei das Griffteil (30) ein Klemmelement (32) umfasst, um das Griffteil (30) abnehmbar am Schaft (10) zu befestigen,
**dadurch gekennzeichnet, dass** das Griffteil (30) einen Griff (31) umfasst, der eine Längsachse aufweist, die im Wesentlichen koaxial zur Werkzeugachse verläuft.

2. Chirurgisches Elektrowerkzeug nach Anspruch 1, wobei die Werkzeugachse in Bezug auf die Längsachse des Schafts (10) um einen Winkel von 60 bis 120 Grad abgewinkelt ist.

3. Chirurgisches Elektrowerkzeug nach einem der vorhergehenden Ansprüche, wobei das Befestigungselement (32) mit einem Abstand vom Kopfteil (20) an dem Schaft (10) befestigt ist.

4. Chirurgisches Elektrowerkzeug nach einem der vorhergehenden Ansprüche, wobei das Griffteil (30) relativ zur Längsachse des Schafts (10) verstellbar ist.

5. Chirurgisches Elektrowerkzeug nach einem der vorhergehenden Ansprüche, wobei das chirurgische Elektrowerkzeug (1) ferner eine Beleuchtungseinrichtung (40) umfasst, um der chirurgischen Eingriffsstelle Licht zuzuführen.

6. Chirurgisches Elektrowerkzeug nach Anspruch 5, wobei das Griffteil (30) dazu eingerichtet ist, zumindest einen Teil der Beleuchtungseinrichtung (40) aufzunehmen.

7. Chirurgisches Elektrowerkzeug nach einem der vorhergehenden Ansprüche, wobei das chirurgische Elektrowerkzeug (1) ferner einen Spiegel (60) und/oder ein Endoskop umfasst, der/das dazu eingerichtet ist, einen Blick auf die chirurgische Eingriffsstelle zu gewähren.

8. Chirurgisches Elektrowerkzeug nach Anspruch 7, wobei der Spiegel (60) und/oder das Endoskop am Kopfteil (20) angeordnet ist/sind.

9. Chirurgisches Elektrowerkzeug nach einem der vorhergehenden Ansprüche, wobei das chirurgische Elektrowerkzeug (10) ferner eine Spüleinrichtung (50) umfasst, um der chirurgischen Eingriffsstelle ein Fluid zuzuführen.

10. Chirurgisches Elektrowerkzeug nach einem der Ansprüche 9, wobei die Spüleinrichtung (50) zumindest teilweise entlang des Schafts (10) und/oder des Griffteils (30) angeordnet ist.

## Revendications

1. Outil chirurgical motorisé (1) comprenant
- une partie de tête (20) ayant un outil (21) sous la forme d'un foret ou d'un embout de tournevis avec un axe d'outil,
- une tige (10) qui est connectée à la partie de tête (20) pour guider la partie de tête (20) jusqu'à un site chirurgical, la tige (10) ayant un axe longitudinal et l'axe d'outil étant en angle par rapport à l'axe longitudinal de la tige (10), et
- une partie de manche (30) pour exercer une pression sur la partie de tête (20) le long de l'axe d'outil,
la partie de manche (30) comprenant un élément de pince (32) pour fixer de façon libérable la partie de manche (30) à la tige (10),
**caractérisé en ce que**
la partie de manche (30) comprend un manche (31) ayant un axe longitudinal qui est sensiblement coaxial avec l'axe d'outil.

2. Outil chirurgical motorisé selon la revendication 1, dans lequel l'axe d'outil est en angle avec un angle de 60 à 120 degrés par rapport à l'axe longitudinal de la tige (10).

3. Outil chirurgical motorisé selon l'une des revendications précédentes, dans lequel l'élément de pince (32) est fixé à la tige (10) à une distance de la partie de tête (20).

4. Outil chirurgical motorisé selon l'une des revendications précédentes, dans lequel la partie de manche (30) est ajustable par rapport à l'axe longitudinal de la tige (10).

5. Outil chirurgical motorisé selon l'une des revendications précédentes, dans lequel l'outil chirurgical motorisé (1) comprend en outre un dispositif d'éclairage (40) pour amener une lumière jusqu'au site chirurgical.

6. Outil chirurgical motorisé selon la revendication 5, dans lequel la partie de manche (30) est adaptée à recevoir au moins une partie du dispositif d'éclairage (40).

7. Outil chirurgical motorisé selon l'une des revendications précédentes, dans lequel l'outil chirurgical motorisé (1) comprend en outre au moins un parmi un miroir (60) et un endoscope configuré pour fournir une vue du site chirurgical.

8. Outil chirurgical motorisé selon la revendication 7, dans lequel le miroir (60) et/ou l'endoscope sont/est agencé(s) au niveau de la partie de tête (20).

9. Outil chirurgical motorisé selon l'une des revendications précédentes, dans lequel l'outil chirurgical motorisé (1) comprend en outre un dispositif de rinçage (50) pour amener un fluide jusqu'au site chirurgical.

10. Outil chirurgical motorisé selon la revendication 9, dans lequel le dispositif de rinçage (50) est agencé au moins partiellement le long d'au moins une parmi la tige (10) et la partie de manche (30).
